(19) Europäisches Patentamt

European Patent Office

Office européen des brevets

(11) **EP 2 419 077 B1**

(12) **EUROPEAN PATENT SPECIFICATION**

(45) Date of publication and mention
of the grant of the patent:
**12.10.2016 Bulletin 2016/41**

(21) Application number: **10708226.5**

(22) Date of filing: **16.03.2010**

(51) Int Cl.:
*A61K 8/73* (2006.01)          *A61Q 5/02* (2006.01)
*A61Q 5/00* (2006.01)          *A61Q 5/12* (2006.01)

(86) International application number:
**PCT/EP2010/053385**

(87) International publication number:
**WO 2010/118925 (21.10.2010 Gazette 2010/42)**

(54) **PROCESS OF TREATING DAMAGED HAIR**

VERFAHREN ZUR BEHANDLUNG VON GESCHÄDIGTEM HAAR

PROCÉDÉ DE TRAITEMENT DE CHEVEUX ABÎMÉS

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **15.04.2009 US 169580 P**

(43) Date of publication of application:
**22.02.2012 Bulletin 2012/08**

(73) Proprietor: **Rhodia Opérations
93306 Aubervilliers (FR)**

(72) Inventor: **MABILLE, Caroline
F-75004 Paris (FR)**

(74) Representative: **Cardon, Flavie
Rhodia Services
Direction Propriété Industrielle
40, rue de la Haie-Coq
FR-93306 Aubervilliers Cedex (FR)**

(56) References cited:
**EP-A1- 0 530 974      EP-A1- 2 022 478
EP-A1- 2 161 016      EP-A2- 0 756 861
AU-B2- 639 351        FR-A1- 2 773 710**

**Description**

FIELD OF THE INVENTION

The present invention concerns a process of treating damaged hair with aqueous compositions that are suitable as shampoos. The compositions comprise a cationic polymer which provides an interesting hair treatment targeting damaged hair.

BACKGROUND OF THE INVENTION

Shampoo compositions are designed to provide certain benefits, including cleaning. These are usually aqueous compositions. Conditioning compositions are designed to provide some further treatment of hair. Some compositions are designed to provide both cleaning and treatment benefits. These are often referred to as 2-in-1 (cleaning and conditioning) shampoos. Some shampoo compositions are designed to provide benefits on virgin hair and/or on damaged hair.

Various compositions have been disclosed in the literature and/or have been made available to consumers. Shampoos typically comprise surfactants, usually anionic surfactants, in the form of micellar aqueous solutions. Using silicones in shampoo compositions is known. Using cationic polymers in shampoos is also known. It is believed that some silicone oil deposits onto hair to provide hair treatment. It is believed that cationic polymers can assist the deposition of silicone oil.

There is a need for improved treatments of damaged hair, typically with aqueous compositions, suitable as shampoo, especially as 2-in-1 shampoos.

When hair comprises damaged hair and virgin hair, there is a need of treatments that target damaged hair, to provide more improvement to damaged hair than to virgin hair.

Prior art document FR 2773710 discloses double modified cationic guar compounds in hair conditioning compositions.

BRIEF SUMMARY OF THE INVENTION

The invention addresses at least one of the needs above with a process for hair treatment comprising the step of applying onto hair composition comprising a cationic polymer and an oil, wherein:

- the hair comprises damaged hair and optionally virgin hair, and
- the cationic polymer is a double modified cationic guar comprising

   a) cationic modifying groups, preferably comprising a group of formula $-N^+Me_3$, and
   b) non ionic modifying groups being $C_3$-$C_{22}$ alkyl or hydroxyalkyl groups.

The process using the composition allows an improved treatment of damaged hair. The invention also allows an improved selectivity of treatment to damaged hair compared to virgin hair. The invention also allows targeting damaged hair. The invention also allows an improved ratio of:

   treatment of damaged hair / treatment of virgin hair.

The improved treatment of damaged hair and/or selectivity or and/or ratio can allow avoiding the use of large quantities of composition. It can allow lowering the frequency of the use of the composition. It can allow avoiding build-up deposition, especially on virgin hair where not much treatment is needed.

It has been found that the double modified guar surprisingly increases the selectivity of treatment to damaged hair.

The invention also concerns composition to be applied onto hair. The invention also concerns a process of preparing the composition. The invention also concerns the use of the composition to treat hair, especially to treat damaged hair. The invention also concerns the use of the double modified guar to increase the selectivity of the treatment for damaged hair.

DETAILED DESCRIPTION OF THE INVENTION

The aqueous composition of the invention comprises various ingredients that are detailed below.

In the present applications, unless otherwise provided, amounts are expressed as active % by weight, as opposed to amounts "as is".

Double modified guar

The composition comprises a double modified guar polymer comprising

a) cationic modifying groups, preferably comprising a group of formula $-N^+Me_3$, and
b) non ionic modifying groups being $C_3$-$C_{22}$ alkyl or hydroxyalkyl groups, preferably hydroxypropyl groups.

Modified guars are polymers obtained by chemically modifying guar gum. The chemical modification is often referred to as derivatization. The modification provides side groups on the guar polymer backbone. The side groups are usually linked to the backbone by ether linkage. The oxygen of the ether linkage corresponds to hydroxyl groups on the guar backbone, reacted for modification. Guar comes from guar gum, the mucilage found in the seed of the leguminous plant Cyamopsis tetragonolobus. The guar seeds used to make guar gum are composed of a pair of tough, non-brittle endosperm sections, hereafter referred to as "guar splits," between which is sandwiched the brittle embryo (germ). After dehulling, the seeds are split, the germ (43-47% of the seed) is removed by screening. The splits typically contain about 78-82% guar gum and minor amounts of some proteinaceous material, inorganic salts, water-insoluble gum, and cell membranes, as well as some residual seed coat and seed embryo.

The water soluble fraction (85%) is called "guaran" or "guar gum" which consists of linear chains of (1,4)-.$\beta$-D mannopyranosyl units-with $\alpha$-D-galactopyranosyl units attached by (1,6) linkages. The ratio of D-galactose to D-mannose is about 1:2. The backbone of guar is herein understood as comprising the mannose and the galactose groups.

Modifications by the cationic groups a) are known by the one skilled in the art. The derivatizing agent can comprise a cationic substituent group that comprises a cationic nitrogen radical, more typically, a quaternary ammonium radical, for example. Typical quaternary ammonium radicals are trialkylammonium radicals, such as trimethylammonium radicals, triethylammonium radicals, tributylammonium radicals, aryldialkylammonium radicals, such as benzyldimethylammonium radicals, and ammonium radicals in which the nitrogen atom is a member of a ring structure, such as pyridinium radicals and imidazoline radicals, each in combination with a counterion, typically a chloride, bromide, or iodide counterion.

In some embodiments, the cationic substituent group is linked to the reactive functional group of the cationizing agent, for example, by an alkylene or oxyalkylene linking group. Suitable cationizing reagents include, for example, epoxy-functional cationic nitrogen compounds, such as, for example, 2,3-epoxypropyltrimethylammonium chloride; chlorohydrin-functional cationic nitrogen compounds, such as, for example, 3-chloro-2-hydroxypropyl trimethylammonium chloride, 3-chloro-2-hydroxypropyl-lauryldimethylammonium chloride, 3-chloro-2-hydroxypropyl-stearyldimethylammonium chloride; and vinyl-, or (meth)acrylamide- functional nitrogen compounds, such as methacrylamidopropyl trimethylammonium chloride. The group a) can be for example hydroxypropyl ammonium. These can be obtained for example by reacting guar gum with compounds such as 2,3-epoxypropyltrimethylammonium chloride or 3-chloro-2-hydroxypropyltrimethylammonium chloride. Some single modified guars are referred to, according to INCI terminology, as Guar Hydroxypropyltrimonium Chloride. Some single modified guars are Jaguar® C17 and Jaguar® C13S, Rhodia.

Modifications by the non ionic groups b) are known by the one skilled in the art. In one embodiment, the guar is reacted with an alkylene oxide derivatizing agent, such as ethylene oxide, propylene oxide, or butylene oxide, under known alkoxylation conditions to add hydroxyalkyl and/or poly(alkyleneoxy) substituent groups to the guar polysaccharide chains. The group b) can be for example hydroxypropyl. These can be obtained for example by reacting guar gum with compounds such as propylene oxide.

Double modified guars are known by the one skilled in the art. Some are referred to, according to INCI terminology, as Hydroxypropyl Guar Hydroxypropyltrimonium Chloride. A double modified guar that can be used is Jaguar® C162, Rhodia.

The degree of modification by groups b) (molar substitution or MS) is preferably between 0.1 and 1.2, preferably of between 0.3 and 0.7. The degree of modification by groups a) (degree of substitution or DS) is preferably between 0.01 and 0.6, preferably between 0.05 and 0.20. This is the case, for example, for the Jaguar@ C162, Rhodia, that has a MS and a DS in these ranges.

The double modified guar can have a weight-average molar mass of at least 10000 g/mol, and more preferably of higher than 100000 g/mol, preferably of higher than 500000 g/mol, for example of from 500000 g/mol to $3 \times 10^6$ g/mol, for example about 1000000 g/mol or even more, depending on their possible degree of polymerization.

Oil

The composition comprises at least an oil. The oil can be for example:

- a silicone oil,
- an oil of mineral origin,
- an oil of vegetal origin, or
- a mixture or association thereof.

The oil is typically present in the composition in the form of dispersed particles or droplets.

The composition can typically comprise from 0.05 to 10% by weight, preferably from 0.1 to 5%; of the oil.

Silicone oils

In one embodiment the composition comprises a silicone oil. Silicone oils are known by the one skilled in the art. These are often referred to as polyorganosiloxanes. In the present application the terms "silicone" or "polyorganosiloxane" can be used indifferently. The term "silicone" or "polyorganosiloxane" is understood to mean any organosiloxane compound comprising alkyl (for example methyl) groups and/or functionalized by groups other than alkyl groups. Silicones can be linear, cyclic, or branched polymers or oligomers of monomeric silicon/oxygen (organosiloxane) monomers, optionally bearing some further functional groups. The polymeric backbone is typically made up of alternating silicon and oxygen atoms. The silicon atoms may carry a wide variety of substituents which can be the same or different. Functional endblocking groups may carry nitrogen or hydroxyl moieties.

The polyorganosiloxane is advantageously (in shampoos and conditioners in particular) a nonvolatile and water-insoluble polyorganosiloxane. It advantageously exhibits a viscosity of between 1000 and 2 000 000 mPa·s, preferably between 5000 and 500 000 mPa·s. The polyorganosiloxane can in particular be a polydimethylorganosiloxane ("PDMS", INCI name: dimethicone), or a polyorganosiloxane exhibiting amine groups (for example, amodimethicone according to the INCI name), quaternary ammonium groups (for example, silicone quaternium-1 to - 10 according to the INCI name), hydroxyl groups (terminal or non terminal), polyoxyalkylene groups, for example polyethylene oxide and/or polypropylene oxide groups (as terminal groups, as blocks within a PDMS chain or as grafts), or several of these groups.

The amount of silicone oil present in the composition can typically be from 0.1 % to 5% by weight, for example from 0.5% to 1.5% or 2% by weight.

The silicone oil (polyorganosiloxane) is preferably present in the composition in an emulsion form (liquid silicone droplets dispersed in the aqueous phase). The silicone oil can be present in the composition in the form of:

- a microemulsion with a particle size of lower than 0,15 $\mu$m,
- an emulsion with a particle size of from 0,15 $\mu$m to lower than 1 $\mu$m, or of from 1 $\mu$m to lower than 1.5 $\mu$m or of from 1.5 $\mu$m to lower than 2 $\mu$m, or from 2 $\mu$m to lower than 2.5 $\mu$m, or from 2.5 $\mu$m to lower than 4 $\mu$m, or from 4 $\mu$m to lower than 10 $\mu$m, or from 10 $\mu$m to lower than 30 $\mu$m, or from 30 $\mu$m to 100 $\mu$m. Sizes herein refer to mean sizes of the droplets.

The droplets of the emulsion can be more or less large in size. Reference may thus be made to microemulsions, to miniemulsions or to macroemulsions. In the present patent application, the term "emulsion" covers in particular all these types of emulsion. Without wishing to be committed to any one theory, it is specified that microemulsions are generally thermodynamically stable systems generally comprising large amounts of emulsifying agents. The other emulsions are generally systems in the non-thermodynamically stable state which retain for a certain time, in the metastable state, the mechanical energy provided during the emulsification. These systems generally comprise lesser amounts of emulsifying agents.

The emulsions can be obtained by mixing the carrier, preferably aqueous carrier, the polyorganosiloxane and generally an emulsifying agent, and then emulsifying. It is possible to speak of in situ emulsification.

The compositions in the emulsion form can also be obtained by mixing the carrier, preferably aqueous carrier, with a pre-prepared emulsion of droplets comprising the polyorganosiloxane in an external phase which is preferably miscible with the cosmetically acceptable carrier, preferably of the same nature as said carrier, preferably an aqueous carrier. This embodiment may be preferred as it is simple to implement. In addition, this embodiment is particularly suitable for the implementation of cosmetic compositions in which the polyorganosiloxane is in the microemulsion form. It is possible to speak of pre-emulsification.

According to a specific embodiment, the emulsion is a microemulsion, the size of the droplets of which is less than 0.15 $\mu$m. In this embodiment, the composition preferably comprises a proportion of emulsifying agent of greater than 10% by weight, preferably at least 15% by weight, with respect to the weight of polyorganosiloxane.

The size of the microemulsion droplets can be measured on an emulsion prepared prior to this introduction into the cosmetic composition by dynamic light scattering (QELS), for example as described below. The equipment used is, for example, composed of a Spectra-Physics 2020 laser, of a Brookhaven 2030 correlator and of the associated computing. As the sample is concentrated, it is diluted in deionized water and filtered through a 0.22 $\mu$m filter in order,

at the end, to be at 2% by weight. The diameter obtained is an apparent diameter. The measurements are carried out at angles of 90° and 135°. For the size measurements, in addition to the conventional analysis by cumulants, the autocorrelation function is run in three ways (the exponential sampling or EXPSAM described by Pr. Pike, the "Non Negatively Constrained Least Squares" or NNLS method and the CONTIN method described by Pr. Provencher) which each give a size distribution weighted by the scattered intensity and not by the weight or the number. The refractive index and the viscosity of the water are taken into account.

According to an advantageous form, the microemulsion is transparent. The microemulsion can, for example, exhibit a transmittance of at least 90%, preferably of at least 95%, at a wavelength of 600 nm, measured, for example, using a Lambda 40 UV-Vis spectrometer at a concentration of 0.5% by weight in water. In this context, the cosmetic composition can advantageously be transparent. It can, for example, exhibit a transmittance of at least 90%, preferably of at least 95%, at a wavelength of 600 nm, measured, for example, using a Lambda 40 UV-Vis spectrometer.

According to another specific embodiment, the emulsion is an emulsion for which the mean size of the droplets is greater than or equal to 0.15 $\mu$m, for example greater than 0.5 $\mu$m, or than 1 $\mu$m, or than 2 $\mu$m, or than 10 $\mu$m, or than 20 $\mu$m, and preferably less than 100 $\mu$m. The size of the droplets can be measured, by optical microscopy and/or laser particle sizing (Horiba LA-910 laser scattering analyzer), on an emulsion prepared prior to its introduction into the cosmetic composition or directly on the cosmetic composition diluted in water. In this embodiment, the composition preferably comprises a proportion of emulsifying agent of less than 10% by weight, with respect to the weight of polyorganosiloxane.

Emulsifying agents of use in the preparation of polyorganosiloxane emulsions are in particular nonionic surfactants, preferably polyalkoxylated surfactants, for example chosen from alkoxylated fatty alcohols, alkoxylated triglycerides, alkoxylated fatty alcohols, alkoxylated sorbitan esters, alkoxylated fatty amines, alkoxylated di(1-phenylethyl)phenols, alkoxylated tri(1-phenylethyl)phenols and alkoxylated alkylphenols, where the number of alkoxy units, more particularly oxyethylene and/or oxypropylene units, is such that the HLB value is greater than or equal to 10.

Mention may be made, among the silicone derivatives which are soluble in the water of the composition, inter alia, of dimethicone copolyols.

As relates to the silicones which are provided in the form of dispersions which are insoluble in the water of the composition, use may suitably be made of water-insoluble and nonvolatile polyorganosiloxanes, among which may be mentioned polyalkylsiloxane, polyarylsiloxane or polyalkylarylsiloxane oils, gums or resins or their water-insoluble functionalized derivatives, or their mixtures, which are nonvolatile.

Said organopolyosiloxanes are regarded as water-insoluble and nonvolatile if their solubility in water is less than 50 g/liter and their intrinsic viscosity is at least 3000 mPa·s at 25°C.

Mention may be made, as examples of water-insoluble and nonvolatile polyorganosiloxanes or silicones, of silicone gums, such as, for example, a diphenyl dimethicone gum, and preferably the polydimethylsiloxanes exhibiting a viscosity at least equal to $6 \times 10^5$ mPa·s at 25°C and more preferably still those with a viscosity of greater than $2 \times 10^6$ mPa·s at 25°C.

According to the invention, the water-insoluble and nonvolatile polyorganosiloxane or silicone occurs in a form dispersed within the cosmetic composition including it.

The water-insoluble and nonvolatile polyorganosiloxane or silicone exists in the form of particles or droplets, the size of which can be chosen according to the nature of the cosmetic composition or the performance desired for said composition. Generally, this size can vary from 0.01 to 70 microns.

In order to facilitate the use thereof, these polyorganosiloxanes can be dispersed or dissolved beforehand in volatile or nonvolatile silicone derivatives of low viscosity and then emulsified in the cosmetic composition.

Mention may be made, among these silicones of low viscosity, of volatile cyclic silicones and polydimethylsiloxanes of low weight.

Use can also be made of functionalized silicone derivatives, such as aminated derivatives, directly in the form of emulsions or starting from a preformed microemulsion. They can be compounds known under the term of aminated silicones or hydroxylated silicones.

Mention is in particular made, as polyorganosiloxanes which can be used, of:

- polyorganosiloxanes comprising -Si(CH$_3$)$_2$O- units and -SiY(CH$_3$)O- units where Y is a -(CH$_2$)$_3$-NH(CH$_2$)$_2$-NH$_2$ or -(CH$_2$)$_3$-NH$_2$ group,
- polyorganosiloxanes comprising -Si(CH$_3$)$_2$O- units and HO-Si(CH$_3$)$_2$O- terminal units and/or -Si(CH$_3$)(OH)O- non terminal units,
- polyorganosiloxanes comprising -Si(CH$_3$)$_2$O- units and -SiY(CH$_3$)O- units where Y is -L$^x$-Z$^x$-Palc where L$^x$ is a divalent connecting group, preferably an alkylene group, Z$^x$ is a covalent bond or a divalent joining group comprising a heteroatom, Palc is a group of formula [OE]$_s$-[OP]$_t$-X', in which OE is a group of formula -CH$_2$-CH$_2$-O-, OP is a group of formula -CH$_2$-CHCH$_3$-O- or -CHCH$_3$-CH$_2$-O-, X' is a hydrogen atom or a hydrocarbon group, s is a mean number greater than 1 and t is a mean number greater than or equal to 0,

- polyorganosiloxanes, the chain of which comprises at least one block comprising units of formula -Si(CH₃)₂O- units and at least one -[OE]ₛ-[OP]ᵣ- block,
- polyorganosiloxanes comprising -Si(CH₃)₂O- units and/or -Si(CH₃)RO- and/or-SiR₂O- and/or R-Si(CH₃)₂O- and/or H₃C-SiR₂O- and/or R-SiR₂O- units, where R, which can be identical or different, is an alkyl group other than a methyl group, an aryl group, an alkylaryl group or an aralkyl group.

Examples of silicone oils that can be used include the following (INCI names) : Amino Bispropyl Dimethicone; Aminopropyl Dimethicone; Aminopropyl Phenyl Trimethicone; Amodimethicone; Amodimethicone Hydroxystearate; Amodimethicone/Silsesquioxane Copolymer; Behentrimonium Dimethicone PEG-8 Phthalate; Bisamino PEG/PPG-41/3 Aminoethyl PG-Propyl Dimethicone; Bis-Aminopropyl Dimethicone; Bis-Aminopropyl/Ethoxy Aminopropyl Dimethicone; Bis-Butyldimethicone Polyglyceryl-3; Bis-Butyloxyamodimethicone/PEG-60 Copolymer; Bis(C13-15 Alkoxy) Hydroxybutamidoamodimethicone; Bis(C13-15 Alkoxy) PG-Amodimethicone; Bis-Hydroxyethoxypropyl Dimethicone Beeswax Esters; Bis-Hydroxyethoxypropyl Dimethicone Isostearate; Bis-Isobutyl PEG-14/Amodimethicone Copolymer; Bis-Isobutyl PEG-15/Amodimethicone Copolymer; Bis-PEG-1 Dimethicone; Bis-PEG-4 Dimethicone; Bis-PEG-8 Dimethicone; Bis-PEG-12 Dimethicone Bis-PEG-20 Dimethicone; Bis-PEG-12 Dimethicone Beeswax; Bis-PEG-12 Dimethicone Candelillate; Bis-PEG-10 Dimethicone/Dimer Dilinoleate Copolymer; Bis-PEG-15 Methyl Ether Dimethicone; Bisphenylhexamethicone; Bis-Phenylpropyl Dimethicone; Bis-(Polyglyceryl-3 Oxyphenylpropyl) Dimethicone; Bis(PPG-7 Undeceneth-21) Dimethicone; Borage Seed Oil PEG-7 Dimethicone Esters; C30-45 Alkyl Cetearyl Dimethicone Crosspolymer; C26-28 Alkyl Dimethicone; Cetearyl Dimethicone/Vinyl Dimethicone Crosspolymer; Cetrimonium Carboxydecyl PEG-8 Dimethicone; Cetyl Triethylmonium Dimethicone PEG-8 Phthalate; Cetyl Triethylmonium Dimethicone PEG-8 Succinate; Cyclohexasiloxane; Cyclomethicone; Cyclopentasiloxane; Cyclophenylmethicone; Cyclotetrasiloxane; Cyclotrisiloxane; DEA PG-Propyl PEG/PPG-18/21 Dimethicone; Dilinoleamidopropyl Dimethylamine, Dimethicone; Dimethicone PEG-7 Phosphate; Dimethicone Hydroxypropyl Trimonium Chloride; Dimethicone/Mercaptopropyl Methicone Copolymer; Dimethicone PEG-15 Acetate; Dimethicone PEG-8 Adipate; Dimethicone PEG-7 Avocadoate; Dimethicone PEG-8 Avocadoate; Dimethicone PEG-8 Beeswax; Dimethicone PEG-8 Borageate; Dimethicone PEG-7 Cocoate; Dimethicone PEG-7 Isostearate; Dimethicone PEG-7 Lactate; Dimethicone PEG-8 Lanolate; Dimethicone PEG-8 Meadowfoamate; Dimethicone PEG-7 Olivate; Dimethicone PEG-8 Olivate; Dimethicone PEG-8 Phosphate; Divinyldimethicone/Dimethicone Copolymer; Dimethicone PEG-7 Phthalate; Dimethicone PEG-8 Phthalate; Dimethicone PEG-7 Succinate; Dimethicone PEG-8 Succinate; Dimethicone PEG-7 Sulfate; Dimethicone PEG-7 Undecylenate; Dimethicone Propyl PG-Betaine; Dimethicone/Silsesquioxane Copolymer; Dimethiconol Arginine; Dimethiconol Cysteine; Dimethiconol Lactate; Dimethiconol Methionine; Dimethiconol Panthenol; Dimethiconol/Silsesquioxane Copolymer; Di-Methoxycinnamidopropyl Ethyldimonium Chloride Ether; Dimethoxysilyl Ethylenediaminopropyl Dimethicone; Dimethylaminopropylamido PCA Dimethicone; Diphenyl Amodimethicone; Diphenylisopropyl Dimethicone; Diphenylsiloxy Phenyl Trimethicone; Glycidoxy Dimethicone; Hexyl Dimethicone; Hydrolyzed Collagen PG-Propyl Dimethiconol; Hydrolyzed Collagen PG-Propyl Methylsilanediol; Hydrolyzed Collagen PG-Propyl Silanetriol; Hydrolyzed Keratin PG-Propyl Methylsilanediol; Hydrolyzed Sesame Protein PG-Propyl Methylsilanediol; Hydrolyzed Silk PG-Propyl Methylsilanediol; Hydrolyzed Silk PG-Propyl Methylsilanediol Crosspolymer; Hydrolyzed Soy Protein/Dimethicone PEG-7 Acetate; Hydrolyzed Soy Protein PG-Propyl Methylsilanediol; Hydrolyzed Vegetable Protein PG-Propyl Silanetriol; Hydrolyzed Wheat Protein/Cystine Bis-PG-Propyl Silanetriol Copolymer; Hydrolyzed Wheat Protein PG-Propyl Methylsilanediol; Hydrolyzed Wheat Protein PG-Propyl Silanetriol; Hydroxypropyldimethicone; Isopolyglyceryl-3 Dimethicone; Isopolyglyceryl-3 Dimethiconol; Lauryl PEG-9 Polydimethylsiloxyethyl Dimethicone; Lauryl Polyglyceryl-3 Polydimethylsiloxyethyl Dimethicone; Linoleamidopropyl PG-Dimonium Chloride Phosphate Dimethicone; Methoxy Amodimethicone/Silsesquioxane Copolymer; Methyleugenyl PEG-8 Dimethicone; Methylsilanol Acetylmethionate; Methylsilanol Elastinate; Methyl Trimethicone; Nylon-611/Dimethicone Copolymer; PCA Dimethicone; PEG-8 Amodimethicone; PEG-3 Dimethicone; PEG-8 Dimethicone; PEG-9 Dimethicone; PEG-10 Dimethicone; PEG-12 Dimethicone; PEG-14 Dimethicone; PEG-17 Dimethicone ; PEG-8 Distearmonium Chloride PG-Dimethicone; PEG-8 Methicone PEG-6 Methicone Acetate; PEG-6 Methyl Ether Dimethicone; PEG-7 Methyl Ether Dimethicone; PEG-8 Methyl Ether Dimethicone; PEG-9 Methyl Ether Dimethicone; PEG-10 Methyl Ether Dimethicone; PEG-11 Methyl Ether Dimethicone; PEG-32 Methyl Ether Dimethicone ; PEG-10 Nonafluorohexyl Dimethicone Copolymer; PEG-12 Methyl Ether Lauroxy PEG-5 Amidopropyl Dimethicone; PEG-8 PG-Coco-Glucoside Dimethicone; PEG/PPG-28/21 Acetate Dimethicone; PEG/PPG-20/22 Butyl Ether Dimethicone; PEG/PPG-22/22 Butyl Ether Dimethicone; PEG/PPG-23/23 Butyl Ether Dimethicone; PEG/PPG-24/18 Butyl Ether Dimethicone; PEG/PPG-27/9 Butyl Ether Dimethicone; PEG/PPG-10/2 Dimethicone; PEG/PPG-20/23 Dimethicone; PEG/PPG-20/22 Methyl Ether Dimethicone; PEG/PPG-24/24 Methyl Ether Glycidoxy Dimethicone; PEG/PPG-10/3 Oleyl Ether Dimethicone; PEG-4 Trifluoropropyl Dimethicone Copolymer; PEG-8 Trifluoropropyl Dimethicone Copolymer; PEG-10 Trifluoropropyl Dimethicone Copolymer; PG-Amodimethicone; Phenyl Methiconol; Phenylpropyldimethylsiloxysilicate; Phenylpropyl Ethyl Methicone; Phenyl Propyl Trimethicone; Phenyl Trimethicone; Polydimethylsiloxy PPG-13 Butyl Ether Silsesquioxane; Polyglyceryl-3 Disiloxane Dimethicone; Polyglyceryl-3 Polydimethylsiloxyethyl Dimethicone; Polysilicone-1; Polysilicone-2; Polysilicone-3; Polysilicone-4; Polysilicone-5;

Polysilicone-6; Polysilicone-7; Polysilicone-8; Polysilicone-10; Polysilicone-13; Polysilicone-14; Polysilicone-18; Polysilicone-18 Cetyl Phosphate; Polysilicone-18 Stearate; PPG-12 Butyl Ether Dimethicone; PPG-12 Dimethicone; PPG-27 Dimethicone; Propoxytetramethyl Piperidinyl Dimethicone; Quaternium-80; Silicone Quaternium-1; Silicone Quaternium-2; Silicone Quaternium-2 Panthenol Succinate; Silicone Quaternium-3; Silicone Quaternium-4; Silicone Quaternium-5; Silicone Quaternium-6; Silicone Quaternium-7; Silicone Quaternium-8; Silicone Quaternium-9; Silicone Quaternium-10; Silicone Quaternium-11; Silicone Quaternium-12; Silicone Quaternium-15; Silicone Quaternium-16; Silicone Quaternium-16/Glycidoxy Dimethicone Crosspolymer; Silicone Quaternium-17; Silicone Quaternium-18; Silicone Quaternium-20; Sodium Dimethicone PEG-7 Acetyl Methyltaurate; Stearalkonium Dimethicone PEG-8 Phthalate; Steardimonium Hydroxypropyl Panthenyl PEG-7 Dimethicone Phosphate Chloride; Steardimonium Hydroxypropyl PEG-7 Dimethicone Phosphate Chloride; Trideceth-9 PG-Amodimethicone; Trifluoropropyl Cyclopentasiloxane; Trifluoropropyl Cyclotetrasiloxane; Trifluoropropyl Dimethicone; Trimethylsiloxyamodimethicone; Trimethylsiloxyphenyl Dimethicone; Gluconamidopropyl Aminopropyl Dimethicone; Cetrimonium Dimethicone PEG-7 Phthalate; Stearyl Aminopropyl Methicone; Myristylamidopropyl Dimethylamine Dimethicone PEG-7 Phosphate; Potassium Dimethicone PEG-7 Panthenyl Phosphate; Sodium PG-Propyldimethicone Thiosulfate Copolymer; Sodium PG-Propyl Thiosulfate Dimethicone; Tetrabutoxypropyl Trisiloxane.

In some preferred embodiments, the silicone oil is:

- a dimethicone
- an amodimethicone
- a dimethiconol,
- a PEG-dimethicone, or
- a mixture or association thereof.

The composition can typically comprise from 0.1 to 5% by weight, for example from 0,2 to 1.5% or from 1.5 to 3.5%, of the silicone oil.

Oil of mineral origin

In one embodiment the oil is an oil of mineral origin. Such compounds are known by the one skilled in the art. Examples of oils of mineral origin that can be used include the following (INCI names):

- Petrolatum,
- Mineral Oil,
- Hydrogenated Polydodecene,
- Hydrogenated Polydecene,
- Polydecene.

Oil of vegetal origin

In one embodiment the oil is an oil of vegetal origin. Such compounds are known by the one skilled in the art. Examples of oils of vegetal origin that can be used include the following (INCI names):

Adansonia Digitata Seed Oil; Alpinia Speciosa Leaf Oil; Argemone Mexicana Oil; Brassica Oleracea Italica (Broccoli) Seed Oil; Calodendrum Capense Nut Oil; Calophyllum Inophyllum Seed Oil; Camellia Chekiangoleosa Seed Oil; Carica Papaya Seed Oil; Cedrus Deodara Seed Oil; Cocos Nucifera (Coconut) Oil; Crambe Abyssinica Seed Oil; Egg Oil; Fragaria Ananassa (Strawberry) Seed Oil; Hydrogenated Camellia Oleifera Seed Oil; Hydrogenated Evening Primrose Oil; Hydrogenated Hazelnut Oil; Hydrogenated Lanolin; Hydrogenated Macadamia Seed Oil; Hydrogenated Rice Bran Oil; Hydrogenated Sesame Seed Oil ; Hydroxylated Jojoba Oil; Isobutylated Lanolin Oil Lanolin Oil; Lesquerella Fendleri Seed Oil; Marmot Oil; Mink Oil; Ocimum Tenuiflorum Oil; Orbignya Cohune Seed Oil; Ostrich Oil; Phormium Tenax Seed Oil; PPG-40-PEG-60 Lanolin Oil; PPG-12-PEG-65 Lanolin Oil; Pongamia Glabra Seed Oil; Pinus Parviflora Seed Oil; Sclerocarya Birrea Seed Oil; Schleichera Trijuga Seed Oil; Simmondsia Chinensis (Jojoba) Seed Oil; Sorbus Aucuparia Seed Oil; Zea Mays (Corn) Oil; Bertholletia Excelsa Seed Oil PEG-8 Esters; Coconut Oil Methylpropanediol Esters; Jojoba Oil PEG-8 Esters; Hydrogenated Castor Oil Behenyl Esters; Hydrogenated Castor Oil Cetyl Esters; Hydrogenated Castor Oil Dimer Dilinoleate; Hydrogenated Castor Oil Stearyl Esters; Hydrogenated Olive Oil Caprylyl Esters; Hydrogenated Olive Oil Cetyl Esters; Hydrogenated Olive Oil Decyl Esters; Hydrogenated Olive Oil Hexyl Esters; Hydrogenated Olive Oil Lauryl Esters; Hydrogenated Olive Oil Myristyl Esters; Hydrogenated Olive Oil Stearyl Esters; Orbignya Oleifera Seed Oil PEG-8 Esters; Passiflora Edulis/Passiflora Incarnata Seed Oils PEG-8 Esters ; Brassica Campestris (Rapeseed) Oil Unsaponifiables; Brassica Oleracea Botrytis (Cauliflower) Oil Unsaponifiables

Butyrospermum Parkii (Shea Butter) Unsaponifiables; Canola Oil Unsaponifiables; Citrus Aurantifolia (Lime) Seed Oil Unsaponifiables; Citrus Aurantium Dulcis (Sweet Orange) Seed Oil Unsaponifiables; Citrus Grandis (Grapefruit) Seed Oil Unsaponifiables; Hydrogenated Apricot Oil Unsaponifiables; Hydrogenated Grapefruit Seed Oil Unsaponifiables; Hydrogenated Lime Seed Oil Unsaponifiables; Hydrogenated Olive Oil Unsaponifiables; Hydrogenated Orange Seed Oil Unsaponifiables; Hydrogenated Sweet Almond Oil Unsaponifiables; Hydrogenated Wheat Germ Oil Unsaponifiables

Helianthus Annuus (Sunflower) Seed Oil Unsaponifiables; Lupinus Albus Oil Unsaponifiables ; Medicago Sativa (Alfalfa) Oil Unsaponifiables; Olea Europaea (Olive) Oil Unsaponifiables; Olea Europaea (Olive) Fruit Unsaponifiables; Persea Gratissima (Avocado) Oil Unsaponifiables ; Prunus Armeniaca (Apricot) Kernel Oil Unsaponifiables S; esamum Indicum (Sesame) Oil Unsaponifiables; Triticum Vulgare (Wheat) Germ Oil Unsaponifiables; Zea Mays (Corn) Oil Unsaponifiables.

Surfactants

The composition can comprise surfactants. It may be a mixture of different surfactants. The surfactants can be anionic, cationic nonionic, amphoteric surfactants, or mixtures or associations. The surfactants included in the composition preferably comprise at least one anionic or cationic surfactant. The surfactants may also comprise amphoteric surfactants (true amphoteric or zwitterionic surfactants), neutral surfactants (nonionic surfactants). The compositions comprising at least one anionic surfactant and at least one amphoteric surfactant are particularly advantageous, especially for reasons of softness. The total amount of surfactants in the composition is between 0 and 30% by weight.

For conditioners compositions, rinsed out or not rinsed out, the surfactant is preferably absent or present in an amount of lower than 5% by weight, and it might preferably be a cationic surfactant.

The surfactant content is advantageously between 10% and 20% by weight. Such composition may comprise salts, for example sodium or ammonium chloride, advantageously in a content of less than 3% by weight. The weight proportion of anionic surfactants relative to the total amount of surfactants is preferably greater than 50% and preferentially greater than 70%.

Anionic surfactants_are known by the one skilled in the art. In a most preferred embodiment, the anionic surfactant comprises an alkylether sulfate and/or an alkyl sulfate.

In one embodiment, the alkyl ether sulfate surfactant comprises one or more compounds according to structure (I):

$$R^1\text{-O-}(C_mH_{2m}O)_n\text{-SO}_3^-X^+ \qquad (I)$$

wherein

$R^1$ is $(C_8\text{-}C_{18})$alkyl or $(C_8\text{-}C_{18})$alkenyl, more typically $(C_{10}\text{-}C_{14})$alkyl,
m is 2, 3, or 4,
n is an integer of from 1 to about 7, more typically from 1 to 8, even more typically from 1 to 6,
$X^+$ is a cation.

In one embodiment, $R^1$ is a branched $(C_8\text{-}C_{18})$alkyl group or a $(C_8\text{-}C_{18})$alkenyl group, more typically a branched $(C_{10}\text{-}C_{16})$alkyl group, such as tridecyl. In one embodiment $R^1$ is a $C_{12}$ group or a lauryl mixture of groups.

In one embodiment, m is 2 or 3, more typically 2. In one embodiment, n is 1, 2, 3, or 4. As used herein, modifying an alkyl or alkenyl group with the suffix "eth" generally indicates the addition of one or more ethylene oxide units, for example, laureth refers to an ethoxylated lauryl group, and the suffix "-n", wherein n is an integer, indicates the number of such ethylene oxide units per group, for example "laureth-3" indicates an ethoxylated lauryl group with 3 ethylene oxide units per lauryl group.

In one embodiment, the alkyl ether sulfate surfactant comprises one or more compounds selected from sodium laureth sulfates, potassium laureth sulfates, magnesium laureth sulfates, ammonium laureth sulfates, monoethanolamine laureth sulfates, diethanolamine laureth sulfates, triethanolamine laureth sulfates, sodium trideceth sulfates, magnesium trideceth sulfates, ammonium trideceth sulfates, monoethanolamine trideceth sulfates, diethanolamine trideceth sulfates, and triethanolamine trideceth sulfates. sodium oleth sulfates, potassium oleth sulfates, magnesium oleth sulfates, ammonium oleth sulfates, monoethanolamine oleth sulfates, diethanolamine oleth sulfates, triethanolamine oleth sulfates.

In one embodiment, the alkyl sulfate surfactant comprises one or more compounds according to structure (II):

$$R^2\text{-O-SO}_3^-3_X^+ \qquad (II)$$

wherein :

R$^2$ is (C$_8$-C$_{18}$)alkyl or (C$_8$-C$_{18}$)alkenyl, and
X$^+$ is a cation.

In one embodiment, R$^2$ is dodecyl, tridecyl, or oleyl.
In one embodiment, the alkyl sulfate surfactant comprises one or more compounds selected from sodium lauryl sulfate, potassium lauryl sulfate, magnesium lauryl sulfate, ammonium lauryl sulfate, monoethanolamine lauryl sulfate, diethanolamine lauryl sulfate, triethanolamine lauryl sulfate, sodium tridecyl sulfate, potassium tridecyl sulfate, magnesium tridecyl sulfate, ammonium tridecyl sulfate, monoethanolamine tridecyl sulfate, diethanolamine tridecyl sulfate, triethanolamine tridecyl sulfate, sodium oleyl sulfate, potassium oleyl sulfate, magnesium oleyl sulfate, ammonium oleyl sulfate, monoethanolamine oleyl sulfate, diethanolamine oleyl sulfate, triethanolamine oleyl sulfate.
Further types of anionic surfactants that can be used, alone or combined with the above sulfates, include:

- linear alkylbenzene sulfonates,
- alpha olefin sulfonates,
- paraffin sulfonates,
- alkyl ester sulfonates,
- alkyl sulfonates,
- alkyl alkoxy carboxylates,
- monoalkyl phosphates,
- dialkyl phosphates,
- sarcosinates,
- isethionates,
- taurates

Particular examples of anionic surfactants that can be used include : ammonium lauryl sulfate, ammonium laureth sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium trideceth sulfate, sodium tridecyl sulfate, ammonium trideceth sulfate, ammonium tridecyl sulfate, sodium cocoyl isethionate, disodium laureth sulfosuccinate, sodium methyl oleoyl taurate, sodium laureth carboxylate, sodium trideceth carboxylate, sodium-monoalkyl phosphates, sodium dialkyl phosphates, sodium lauryl sarcosinate, lauroyl sarcosine, cocoyl sarcosinate, ammonium cocyl sulfate, sodium cocyl sulfate, potassium cocyl sulfate, monoethanolamine cocyl sulfate, sodium tridecyl benzene sulfonate, sodium dodecyl benzene sulfonate, and branched anionic surfactants, such as sodium trideceth sulfate, sodium tridecyl sulfate, ammonium trideceth sulfate, and ammonium tridecyl sulfate.
The cation of any anionic surfactant is typically sodium but may alternatively be potassium, lithium, calcium, magnesium, ammonium, or an alkyl ammonium having up to 6 aliphatic carbon atoms including isopropylammonium, monoethanolammonium, diethanolammonium, and triethanolammonium. Ammonium and ethanolammonium salts are generally more soluble that the sodium salts. Mixtures of the above cations may be used.
In some embodiments the composition comprises an amphoteric or zwitterionic surfactant. Examples of amphoteric or zwitterionic surfactants that can be used include the following (INCI names):

Almondamidopropyl Betaine; Apricotamidopropyl Betaine; Avocadamidopropyl Betaine; Babassuamidopropyl Betaine; Behenamidopropyl Betaine; Behenyl Betaine; Behenyl Hydroxyethyl Imidazoline; Canolamidopropyl Betaine; Capryl/Capramidopropyl Betaine ; Capryl Hydroxyethyl Imidazoline; Caprylyl Hydroxyethyl Imidazoline; Cetyl Betaine; Coco-Betaine; Cocamidoethyl Betaine; Cocamidopropyl Betaine; Cocamidopropyl Hydroxysultaine; Coco-Hydroxysultaine; Coco/Oleamidopropyl Betaine; Cupuassuamidopropyl Betaine; Coco-Sultaine; DEA-Cocoamphodipropionate; DEA-Lauraminopropionate; Decyl Betaine; Cocoyl Hydroxyethyl Imidazoline; Decyl Mercaptomethylimidazole; Disodium Caproamphodiacetate; Disodium Caproamphodipropionate; Disodium Capryloamphodiacetate; Disodium Capryloamphodipropionate; Disodium Cocaminopropyl Iminodiacetate; Disodium Cocoamphocarboxyethylhydroxypropylsulfonate; Disodium Cocoamphodiacetate; Disodium Cocoamphodipropionate; Disodium Dicarboxyethyl Cocopropylenediamine; Disodium Isostearoamphodiacetate; Disodium Isostearoamphodipropionate; Disodium Laureth-5 Carboxyamphodiacetate; Disodium Lauriminodiacetate; Disodium Lauriminodipropionate; Disodium Lauriminodipropionate Tocopheryl Phosphates; Disodium Lauroamphodiacetate; Disodium Lauroamphodipropionate; Disodium Oleoamphodipropionate; Disodium PPG-2-Isodeceth-7 Carboxyamphodiacetate; Disodium Steariminodipropionate; Disodium Stearoamphodiacetate; Disodium Tallowamphodiacetate; Disodium Tallowiminodipropionate; Disodium Wheatgermamphodiacetate; Glutamylamidoethyl Imidazole; Hydrogenated Tallow Betaine; Hydroxyethyl Carboxymethyl Cocamidopropylamine; Hydroxyethyl Diphenyl Imidazoline; Isostearamidopropyl Betaine; Isostearyl Hydroxyethyl Imidazoline; Lauramidobutyl Guanidine Acetate; Lauramidobutyl Guanidine HCl; Lauramidopropyl Betaine; Lauramidopropyl Hydroxysultaine; Lauryl Betaine; Lauryl Hydroxyethyl Imidazoline; Lauryl Hydroxysultaine; Methoxycinnamidopropyl Hydroxysultaine; Milkamidopropyl Betaine

Minkamidopropyl Betaine; Myristamidopropyl Betaine; Myristamidopropyl Hydroxysultaine; Myristyl Betaine Myristyl Hydroxyethyl Imidazoline; Oleamidopropyl Betaine; Oleamidopropyl Hydroxysultaine; Oleyl Betaine; Oleyl; Hydroxyethyl Imidazoline; Olivamidopropyl Betaine; Palmamidopropyl Betaine; Palmitamidopropyl Betaine; Palm Kernelamidopropyl Betaine; Ricinoleamidopropyl Betaine; Sesamidopropyl Betaine; Sodium C12-15 Alkoxypropyl Iminodipropionate; Sodium Caproamphoacetate; Sodium Caproamphohydroxypropylsulfonate; Sodium Caproamphopropionate; Sodium Capryloamphoacetate; Sodium Capryloamphohydroxypropylsulfonate; Sodium Capryloamphopropionate; Sodium Cocaminopropionate; Sodium Cocoabutteramphoacetate; Sodium Cocoamphoacetate; Sodium Cocoamphohydroxypropylsulfonate; Sodium Cocoamphopropionate; Sodium Cornamphopropionate; Sodium Dicarboxyethylcoco Phosphoethyl Imidazoline; Sodium Isostearoamphoacetate; Sodium Isostearoamphopropionate; Sodium Lauraminopropionate; Sodium Lauriminodipropionate; Sodium Lauroamphoacetate; Sodium Lauroamphohydroxypropylsulfonate; Sodium Lauroampho PG-Acetate Phosphate; Sodium Lauroamphopropionate; Sodium Myristoamphoacetate; Sodium Oleoamphoacetate; Sodium Oleoamphohydroxypropylsulfonate; Sodium Oleoamphopropionate; Sodium Olivamphoacetate; Sodium Palmamphoacetate; Sodium Peanutamphoacetate; Sodium Ricinoleoamphoacetate; Sodium Sesamphoacetate; Sodium Stearoamphoacetate; Sodium Stearoamphohydroxypropylsulfonate; Sodium Stearoamphopropionate; Sodium Sunflowerseedamphoacetate; Sodium Sweetalmondamphoacetate; Sodium Tallamphopropionate

Sodium Tallowamphoacetate; Sodium Undecylenoamphoacetate; Sodium Undecylenoamphopropionate; Sodium Wheat Germamphoacetate; Soyamidopropyl Betaine; Soy Hydroxyethyl Imidazoline; Stearamidopropyl Betaine; Stearyl Betaine Stearyl Hydroxyethyl Imidazoline; TEA-Lauraminopropionate; TEA-Myristaminopropionate; Tall Oil Benzyl Hydroxyethyl Imidazolinium Chloride; Tall Oil Hydroxyethyl Imidazoline; Tallowamidopropyl Betaine; Tallowamidopropyl Hydroxysultaine; Tallow Betaine; Tallow Dihydroxyethyl Betaine; Tallow Hydroxyethyl Imidazoline; Undecylenamidopropyl Betaine; Wheat Germamidopropyl Betaine

Most preferred amphoteric or zwitterionic surfactant include betaines; such as alkylbetaïnes or alkylamidopropyl betaïnes, or imidazoline derivatives, such as alkylamphoacetates or alkylamphodiacetates, where the alkyl is a C8-C22 alkyl group or mixture or C8-C22 alkyl group, optionally comprising insaturations.

In some embodiments the composition comprises non ionic surfactant. Examples of non ionic surfactants that can be used include the following:

- alkoxylated fatty alcohols
- alkoxylated triglycerides
- alkoxylated fatty acids
- alkoxylated sorbitan esters
- alkoxylated fatty amines
- alkoxylated bis(1-phenylethyl)phenols
- alkoxylated tris(1-phenylethyl)phenols
- alkoxylated alkylphenols
- products resulting from the condensation of ethylene oxide with a hydrophobic compound resulting from the condensation of propylene oxide with propylene glycol, such as the Pluronic products sold by BASF;
- products resulting from the condensation of ethylene oxide with the compound resulting from the condensation of propylene oxide with ethylenediamine, such as the Tetronic products sold by BASF;
- alkylpolyglycosides, for instance those described in US 4 565 647;
- fatty acid amides, for example of $C_8$-$C_{20}$.

Examples of useful compositions that may be mentioned include:

- the "sodium" compositions for shampoos typically comprising 12% to 16% by weight of sodium alkyl ether sulfate (for example sodium lauryl ether sulfate "SLES") or a mixture of sodium alkyl ether sulfate and of sodium alkyl sulfate (for example sodium lauryl sulfate "SLS"), 1% to 3% of an amphoteric surfactant (for example cocoamidopropyl betaine "CAPB"), 0.5% to 2% of a salt (for example sodium chloride);
- the "ammonium" compositions for shampoos typically comprising 12% to 16% by weight of ammonium alkyl ether sulfate (for example ammonium lauryl ether sulfate "ALES") or of a mixture of ammonium alkyl ether sulfate and of ammonium alkyl sulfate (for example ammonium lauryl sulfate "ALS"), 1% to 3% of an amphoteric surfactant (for example cocoamidopropyl betaine "CAPB"), 0 to 2% of a salt (for example ammonium chloride).

Other ingredients

The composition is an aqueous composition. Thus it comprises water. All or a part of water can be introduced as part of the products used to make the composition (if these products comprise some water, the active % being thus of less than 100%). All or a part of water can be introduced independently, at various stage, in amounts such the composition is completed to 100%.

The composition might comprise some further ingredients. Further ingredients can impart some further specific properties to the composition. The one skilled in the art knows such ingredients and/or properties that can be associated to these and/or appropriate amounts.

In one embodiment the composition comprises at least one cationic or ampholytic polymer, different form the double modified guar (also referred to as "further cationic or ampholytic polymer"). The composition can comprise a mixture or association of several further cationic or ampholytic polymers. Such compounds can further assist in oil deposition. They might also provide some conditioning effects. In some embodiments these compounds can also help in increasing the viscosity and/or the stability of the composition. They can for example enhance the appearance and feel of hair, increase hair body or suppleness, facilitate styling, improve gloss or sheen and improve the texture of hair that has been damaged by chemical or physical action. They can provide anti-static effect, in altering the static electrical properties of hair.

At least one further cationic or ampholytic polymer can be for example

c1) a modified polysaccharide, for example a cationic cellulose or a cationic guar,
c2) a synthetic cationic polymer, for example polymers comprising units having a quaternary ammonium group or a tertiary ammonium group, and optionally neutral units
c3) a synthetic ampholytic copolymer, for example polymers comprising units having a quaternary ammonium group or a tertiary ammonium group, units having an anionic (usually acidic) group and optionally neutral units, or a mixture or association thereof. Such compounds are known by the one skilled in the art. Examples of useful compounds include (INCI names) polyquaternium polymers from polyquaternium-1 to polyquaternium-95; Polybeta-Alanine; Polyepsilon-Lysine; Polylysine; PEG-8/SMDI Copolymer; PPG-12/SMDI Copolymer; PPG-51/SMDI Copolymer; PPG-7/Succinic Acid Copolymer; IPDI/PEG-15 Cocamine Copolymer; IPDI/PEG-15 Cocamine Copolymer Dimer Dilinoleate; IPDI/PEG-15 Soyamine Copolymer; IPDI/PEG-15 Soyamine Oxide Copolymer; IPDI/PEG-15 Soyethonium Ethosulfate Copolymer; Polyquaternium-4/Hydroxypropyl Starch Copolymer; Cassia Hydroxypropyltrimonium Chloride; Chitosan Hydroxypropyltrimonium Chloride; Dextran Hydroxypropyltrimonium Chloride; Galactoarabinan Hydroxypropyltrimonium Chloride; Ginseng Hydroxypropyltrimonium Chloride; Guar Hydroxypropyltrimonium Chloride; Locust Bean Hydroxypropyltrimonium Chloride; Starch Hydroxypropyltrimonium Chlorid; Hydroxypropyltrimonium Hydrolyzed Wheat Starch; Hydroxypropyltrimonium Hydrolyzed Corn Starch; Hydroxypropyl Oxidized Starch PG-Trimonium Chloride; Tamarindus Indica Hydroxypropyltrimonium Chloride; Polyacrylamidopropyltrimonium Chloride; Polymethacrylamidopropyltrimonium Chloride; Polymethacrylamidopropyltrimonium Methosulfate; Propyltrimoniumchloride Methacrylamide/Dimethylacrylamide Copolymer; Acrylamide/Ethalkonium Chloride Acrylate Copolymer; Acrylamide/Ethyltrimonium Chloride Acrylate/Ethalkonium Chloride Acrylate Copolymer; Acrylates/Carbamate Copolymer; Adipic Acid/Methyl DEA Crosspolymer; Diethylene Glycol/DMAP Acrylamide/PEG-180/HDI Copolymer; Dihydroxyethyl Tallowamine/IPDI Copolymer; Dimethylamine/Ethylenediamine/Epichlorohydrin Copolymer; HEMA Glucoside/Ethylmethacrylate Trimonium Chloride Copolymer; Hydrolyzed Wheat Protein/PEG-20 Acetate Copolymer; Hydrolyzed Wheat Protein/PVP Crosspolymer; Ethyltrimonium Chloride Methacrylate/Hydroxyethylacrylamide Copolymer.

The composition can typically comprise from 0.05 to 5% by weight, for preferably from 0.1 to 1.5%, for example from 0.1 to 0.4% or from 0.4 to 1%, of the further cationic or ampholytic polymer.

In one embodiment the composition comprises a rheology modifier. These are known by the one skilled in the art. Rheology modifiers can be used to adjust the viscosity and/or the stability of the composition. Some useful and popular rheology modifiers are for example polyacrylates polymers (including copolymers). Some useful rheology modifiers are mineral such as clays. Some useful and popular rheology modifiers are natural gums or chemical derivatives thereof.

It is mentioned that some compounds might have rheology modifying effect while being cationic or amphoteric polymers.

Examples of rheology modifiers of natural origin, optionally chemically modified, that can be used include (INCI names):

Agar; Agarose; Alcaligenes Polysaccharides; Algin; Alginic Acid; Ammonium Alginate; Amylopectin; Arachis Hypogaea (Peanut) Flour; Ascorbyl Methylsilanol Pectinate; Astragalus Gummifer Gum; Attapulgite; Avena Sativa (Oat) Kernel Flour; Butoxy Chitosan; Bentonite; Biotite; Caesalpinia Spinosa Gum; Calcium Alginate; Calcium Carboxymethyl Cellulose; Calcium Carrageenan; Calcium Lignosulfonate; Calcium Starch Octenylsuccinate; C12-16 Alkyl PEG-2 Hydroxypropyl Hydroxyethyl Ethylcellulose; Carboxybutyl Chitosan; Carboxymethyl Cellulose Acetate Butyrate; Carboxymethyl Chitin; Carboxymethyl Chitosan; Carboxymethyl Dextran; Carboxymethyl Hydroxyethylcellulose; Carboxymethyl Hydroxypropyl Guar; Carnitine; Cassia Gum; Cellulose Acetate Propionate Carboxylate; Cellulose Gum; Ceratonia Siliqua Gum; Cetyl Hydroxyethylcellulose; Chitosan Lauramide Succinamide; Cobalt DNA; Corallina Officinalis Powder; Croscarmellose; Cyamopsis Tetragonoloba (Guar) Gum; Dehydroxanthan Gum; Dextrin; Dihydrogenated Tallow Benzylmonium Hectorite; Disteardimonium Hectorite; Distarch Phosphate Acetate; Gelatin; Gellan Gum; Glyceryl Alginate; Glycine Soja (Soybean) Flour; Grateloupia Livida Powder; Guar Hydroxypropyltrimonium Chloride; Hectorite; Helianthus Annuus (Sunflower) Seed Flour; Hydrogenated Isocetyl Olivate; Hydrogenated Lecithin; Hydrogenated Potato Starch; Hydrolyzed Corn Starch Hydroxyethyl Ether; Hydroxybutyl Methylcellulose; Hydroxyethylcellulose; Hydroxyethyl Chitosan; Hydroxyethyl Ethylcellulose; Hydroxypropylcellulose; Hydroxypropyl Chitosan; Hydroxypropyl Guar; Hydroxypropyl Methylcellulose; Hydroxypropyl Methylcellulose Stearoxy Ether; Hydroxypropyl Starch; Hydroxypropyl Starch Phosphate; Hydroxypropyltrimonium Maltodextrin Crosspolymer; Hydroxypropyl Xanthan Gum; Locust Bean Gum; Levan; Linum Usitatissimum (Linseed) Seed Flour; Magnesium Alginate; Maltodextrin; Methylcellulose; Methyl Ethylcellulose; Methyl Hydroxyethylcellulose; Microcrystalline Cellulose; Natto Gum; Nitrocellulose; Nonoxynyl Hydroxyethylcellulose; Pectin; Perlite; Polyvinyl Alcohol-Potassium Alginate; Potassium Carrageenan; Potato Starch Modified; Propylene Glycol Alginate; Quaternium-18/Benzalkonium Bentonite; Quaternium-18 Hectorite; Quaternium-90 Bentonite; Rhizobian Gum; Sclerotium Gum; Sodium Arachidate; Sodium Carboxymethyl Chitin; Sodium Carboxymethyl Dextran; Sodium Carboxymethyl Beta-Glucan; Sodium Carboxymethyl Starch; Sodium Carrageenan; Sodium Cellulose Sulfate; Sodium Cyclodextrin Sulfate; Sodium Dextran Sulfate; Sodium Dimaltodextrin Phosphate; Sodium Hydroxypropyl Starch Phosphate; Sodium Isooctylene/MA Copolymer; Sodium Polygamma-Glutamate; Sodium Stearoxy PG-Hydroxyethylcellulose Sulfonate; Solanum Tuberosum (Potato) Starch; Starch Acetate/Adipate; Starch/Acrylates/Acrylamide Copolymer; Starch Hydroxypropyltrimonium Chloride; Stearalkonium Bentonite; Stearalkonium Hectorite; Sterculia Urens Gum; Sucrose Benzoate/Sucrose Acetate Isobutyrate/Butyl Benzyl Phthalate Copolymer; Tamarindus Indica Seed Gum; Tapioca Starch; Tara Gum; TEA-Alginate; Triticum Vulgare (Wheat) Starch; Welan Gum; Xanthan Gum; Yeast Beta-Glucan; Yeast Polysaccharides.

Examples of synthetic rheology modifyiers that can be used include (INCI names): Acrylamide/Ethalkonium Chloride Acrylate Copolymer; Acrylamide/Ethyltrimonium Chloride Acrylate/Ethalkonium Chloride Acrylate Copolymer; Acrylamides Copolymer; Acrylamide/Sodium Acrylate Copolymer; Acrylamide/Sodium Acryloyldimethyltaurate Copolymer; Acrylates/Acetoacetoxyethyl Methacrylate Copolymer; Acrylates/Beheneth-25 Methacrylate Copolymer; Acrylates/C10-30 Alkyl Acrylate Crosspolymer; Acrylates/Ceteth-20 Itaconate Copolymer; Acrylates/Ceteth-20 Methacrylate Copolymer; Acrylates/Laureth-25 Methacrylate Copolymer; Acrylates/Palmeth-25 Acrylate Copolymer; Acrylates/Palmeth-25 Itaconate Copolymer; Acrylates/Steareth-50 Acrylate Copolymer; Acrylates/Steareth-20 Itaconate Copolymer; Acrylates/Steareth-20 Methacrylate Copolymer; Acrylates/Stearyl Methacrylate Copolymer; Acrylates/Vinyl Isodecanoate Crosspolymer; Acrylates/Vinyl Neodecanoate Crosspolymer; Acrylates Copolymer; Acrylates/Methoxy PEG-15 Methacrylate Copolymer; Acrylates/Steareth-20 Methacrylate Crosspolymer; Acrylates/Vinyl Isodecanoate Crosspolymer; Acrylates/VP Copolymer; Acrylic Acid/Acrylamidomethyl Propane Sulfonic Acid Copolymer; Acrylic Acid/Acrylonitrogens Copolymer; Acrylic Acid/Stearyl Methacrylate/Dimethicone Methacrylate Copolymer; Acrylic Acid/VP Crosspolymer; Adipic Acid/Methyl DEA Crosspolymer; Ammonium Acrylates/Acrylonitrogens Copolymer; Ammonium Acrylates Copolymer; Ammonium Acryloyldimethyltaurate/Steareth-8 Methacrylate Copolymer; Ammonium Acryloyldimethyltaurate/Vinyl Formamide Copolymer; Ammonium Acryloyldimethyltaurate/VP Copolymer; Ammonium Polyacryloyldimethyl Taurate; Ammonium Styrene/Acrylates Copolymer; Ammonium VA/Acrylates Copolymer; Bis-Butyldimethicone Polyglyceryl-3; Bis-Decyltetradecanyl IPDI/PEG-795 Copolymer; Bis-Stearyl IPDI/PEG-795 Copolymer; C4-24 Alkyl Dimethicone/Divinyldimethicone Crosspolymer; C6-14 Perfluoroalkylethyl Acrylate/HEMA Copolymer; Calcium Potassium Carbomer; Carbomer; Corn Starch/Acrylamide/Sodium Acrylate Copolymer; Decyltetradeceth-200 Behenate; Decyltetradeceth-200 Isostearate; Diallyloxyneohexyl Zirconium Tridecanoate; Dimethicone Crosspolymer; Dimethicone/PEG-10 Crosspolymer; Dimethicone/PEG-15 Crosspolymer ; Dimethiconol/Stearyl Methicone/Phenyl Trimethicone Copolymer; Dimethylol Urea/Phenol/Sodium Phenolsulfonate Copolymer; Dipentaerythrityl Pentaisostearate; Disodium Methylene Dinaphthalenesulfonate; Ditrimethylolpropane Isostearate/Sebacate; Ditrimethylolpropane Triethylhexanoate; Dimethicone Propyl PG-Betaine; Dimethylacrylamide/Acrylic Acid/Polystyrene ; Dimethylacrylamide/Sodium Acryloyldimethyltaurate Crosspolymer; Disteareth-75 IPDI; Disteareth-100 IPDI; DMAPA Acrylates/Acrylic Acid/Acrylonitrogens Copolymer; Erythrityl Triethylhexanoate; Ethyl Methacrylate Copolymer; Ethylene/MA Copolymer; Ethylene/Sodium Acrylate Copolymer; Ethylene/VA Copolymer; Ethylhexyl Hydroxystearoyl Hydroxystearate; Ethyl Trisiloxane; Glass; Glass Beads; Glyceryl Acrylate/Acrylic Acid Copolymer; Hydroxyethyl Acrylate/Sodium Acryloyldimethyl Taurate Copolymer; Hydroxyethyl PEI-1000; Hydroxyethyl PEI-1500; Hydroxypropyl Ethylenediamine Carbomer; Isobutylene/MA Copolymer; Isobutylene/Sodium Maleate Copolymer; Isopolyglyceryl-3 Dimethicone; Isopolyglyceryl-3 Dimethiconol; Isopropyl Ester of PVM/MA Copolymer; Lauryl PEG-9 Polydimethylsiloxyethyl Dimethicone; Lauryl Polyglyceryl-3 Polydimethylsiloxyethyl Dimethicone; Methacryloyl Ethyl Betaine/Acrylates Copolymer; Methoxy PEG-17/Dodecyl Glycol Copolymer; Methoxy PEG-22/Dodecyl Glycol Copolymer; Methoxy PEG-114/Polyepsilon Caprolactone; Octadecene/MA Copolymer; PEG-800; PEG-Crosspolymer; PEG-150/Decyl Alcohol/SMDI Copolymer; PEG-175 Diisostearate; PEG-150 Distearate; PEG-190 Distearate; PEG-15 Glyceryl Tristearate; PEG-140 Glyceryl Tristearate; PEG-240/HDI Copolymer Bis-Decyltetradeceth-20 Ether; PEG-200 Hydrgenated Glyceryl Palmate; PEG-100/IPDI Copolymer; PEG-180/Laureth-50/TMMG Copolymer; PEG-10/Lauryl Dimethicone Crosspolymer; PEG-15/Lauryl Dimethicone Crosspolymer; PEG-2M; PEG-5M; PEG-7M; PEG-9M; PEG-14M; PEG-20M; PEG-23M; PEG-25M; PEG-45M; PEG-65M; PEG-90M; PEG-115M; PEG-160M; PEG-180M ; PEG-120 Methyl Glucose Trioleate; PEG-180/Octoxynol-40/TMMG Copolymer; PEG-150 Pentaerythrityl Tetrastearate; PEG/PPG-120/10 Trimethylolpropane Trioleate Octyldodecyl/PPG-3 Myristyl Ether Dimer Dilinoleate; PEG-18 Castor Oil Dioleate; PEG-150/Decyl Alcohol/SMDI Copolymer; PEG-12 Dimethicone Crosspolymer; PEG-150/Stearyl Alcohol/SMDI Copolymer; PEI-7; PEI-10; PEI-15; PEI-30; PEI-35; PEI-45; PEI-250; PEI-275; PEI-700; PEI-1000; PEI-1400; PEI-1500; PEI-1750; PEI-2500; PEI-14M; Pentafluoropropane; Perfluorononyl Octyldodecyl Glycol Meadowfoamate; Phosphonobutanetricarboxylic Acid; Polyacrylamidomethylpropane Sulfonic Acid; Polyacrylate-3; Polyacrylate-10; Polyacrylate-11; Polyacrylic Acid; Polycaprolactone; Polycyclopentadiene; Polyester-5; Polyether-1; Polyethylacrylate; Polyethylene/Isopropyl Maleate/MA Copolyol; Polyglycerin-20; Polyglycerin-40; Polyglyceryl-3 Disiloxane Dimethicone; Polyglyceryl-3 Polydimethylsiloxyethyl Dimethicone; Polyglyceryl-4 Isostearate/Laurate; Polyhydroxystearic Acid; Polymethacrylic Acid; Polyoxymethylene Cyanoguanidine Urea; Polyperfluoroethoxymethoxy PEG-2 Phosphate; Polyquaternium-52; Polyvinyl AlcoholPotassium Alginate; Polyvinyl Imidazolinium Acetate; Polyvinyl Methyl Ether; Potassium Aluminum Polyacrylate; Potassium Carbomer; Potassium Polyacrylate ; PPG-3 Myristyl Ether Neoheptanoate; PPG-14 Laureth-60 Hexyl Dicarbamate; PPG-14 Laureth-60 Isophoryl Dicarbamate; PPG-14 Palmeth-60 Hexyl Dicarbamate; PVM/MA Copolymer; PVP; PVP/VA/Itaconic Acid CopolymerPEG-150/Stearyl Alcohol/SMDI Copolymer; PVP/Decene Copolymer; Ricinoleic Acid/Adipic Acid/AEEA Copolymer; Silica; Silica Dimethicone Silylate; Silica Dimethyl Silylate; Silica Silylate; Sodium Acrylates/Acrolein Copolymer; Sodium Acrylates/Acrylonitrogens Copolymer; Sodium Acrylates Copolymer; Sodium Acrylates Crosspolymer; Sodium Acrylate/Sodium Acrylamidomethylpropane Sulfonate Copolymer; Sodium Acrylate/Sodium Acryloyldimethyl Taurate/Acrylamide Copolymer; Sodium Acrylate/Sodium Acryloyldimethyl Taurate Copolymer; Sodium Acrylates/Vinyl Isodecanoate Crosspolymer; Sodium Acrylate/Vinyl Alcohol Copolymer; Sodium Acrylic Acid/MA Copolymer; Sodium Acryloyldimethyl Taurate/Acrylamide/VP Copolymer; Sodium Carbomer; Sodium C4-12 Olefin/Maleic Acid Copolymer; Sodium Glycereth-1 Polyphosphate; Sodium Isooctylene/MA Copolymer; Sodium Magnesium Fluorosilicate; Sodium Polyacrylate; Sodium Polyacrylate Starch; Sodium Polyacryloyldimethyl Taurate; Sodium Polymethacrylate; Sodium Polystyrene Sulfonate; Sodium Styrene/Acrylates Copolymer; Sodium Tauride Acrylates/Acrylic Acid/Acrylonitrogens Copolymer; Steareth-60 Cetyl Ether; Steareth-100/PEG-136/HDI Copolymer; Stearyl/PPG-3 Myristyl Ether Dimer Dilinoleate; Stearylvinyl Ether/MA Copolymer; Styrene/Acrylates/Acrylonitrile Copolymer; Styrene/Acrylates/Ammonium Methacrylate Copolymer; Styrene/MA Copolymer; TEA-Carbomer; Tosylamide/Epoxy Resin; Tosylamide/Formaldehyde Resin; Tribenzoyl Triricinolein; Tromethamine Acrylates/Acrylonitrogens Copolymer; VP/Dimethylaminoethylmethacrylate Copolymer; VP/Eicosene Copolymer; VP/Hexadecene Copolymer; VP/VA Copolymer.

In one embodiment the composition comprises an opacifier and/or pearlizer. These are known by the one skilled in the art. It is mentioned that some of these compounds, such as EGDS, can also be used as rheology modifiers. Opacifying and Pearlizing Agents are ingredients deliberately added to cosmetic products to reduce their clear or transparent appearance, and provide some shine and pearlescent appearance.

Examples of opacifiers and/or pearlizers that can be used include (INCI names):

Acrylates/PEG-10 Maleate/Styrene Copolymer; Allyl Methacrylates Crosspolymer; Alumina; Aluminum Hydroxide; Aluminum Silicate; Aluminum Zinc Oxide; Arachidic Acid; Attapulgite; Barium Sulfate; Behenamide; Behenic Acid; Bentonite; Calamine; Calcium Carbonate; Calcium Silicate; Calcium Sulfate; Calcium Sulfate Hydrate; Cellulose Succinate; Cerium Oxide; Cetearyl Alcohol; Cetyl Alcohol; Chalk; Charcoal Powder; Corchorus Capsularis Powder; Corn Acid; DEA-Styrene/Acrylates/DVB Copolymer; Diatomaceous Earth; Dicalcium Phosphate; Dicalcium Phosphate Dihydrate; Dolomite; Erucamide; Fuller's Earth; Glycol Dibehenate; Glycol Dioleate; Glycol Distearate; Glycol Ditallowate; Glycol Hydroxystearate; Glycol Montanate; Glycol Palmitate; Glycol Stearate; Gossypium Herbaceum (Cotton) Powder; Guanine; Hectorite; Hydrated Silica; Hydrogenated Coconut Acid; Hydrogenated Menhaden Acid; Hydrogenated Palm Acid; Hydrogenated Tallow Acid; Hydrogenated Tallow Amide; Hydroxyethyl Acrylate/Sodium Acryloyldimethyl Taurate Copolymer; Hydroxyethyl Stearamide-MIPA; Hydroxystearyl Cetyl Ether; Iron Powder Kaolin; Lauryl Laurate; Linoleamide; Lithium Stearate; Magnesium Aluminum Silicate; Magnesium Carbonate; Magnesium Oxide; Magnesium Silicate; Magnesium Trisilicate; Mica; Montmorillonite; Moroccan Lava Clay; Myristic Acid; Nephrite Powder; Nylon-6; Nylon 6/12; Nylon-11; Nylon-12; Nylon-66; Palm Acid; Palmitic Acid; Palm Kernel Acid; PEG-3 Distearate; PEG-4 Distearyl Ether; Pisum Sativum (Pea) Starch; Polyacrylate-4; Polyacrylate-10; Polyacrylate-11; Polydodecanamideaminium Triazadiphenylethenesulfonate; Polymethylsilsesquioxane; Polyphenylsilsesquioxane; Polyquaternium-62; Polysilicone-12; Potassium Cellulose Succinate; Propylene Glycol Distearate; Pueraria Lobota Starch; Pyrophyllite; Rubber Latex; Sapphire Powder; Sasa Senanensis Leaf Powder; Silica; Silicon/Titanium/Cerium/Iron Oxides; Silicon/Titanium/Cerium/Zinc Oxides; Sodium Acrylate/Sodium Acryloyldimethyl Taurate/Acrylamide Copolymer; Sodium Acrylate/Sodium Acryloyldimethyl Taurate Copolymer; Sodium Acryloyldimethyl Taurate/Acrylamide/VP Copolymer; Sodium Magnesium Fluorosilicate; Sodium Methacrylate/Styrene Copolymer; Sodium Styrene/Acrylates/Divinylbenzene Copolymer; Sodium Styrene/Acrylates/PEG-10 Dimaleate Copolymer; Sodium Styrene/PEG-10 Maleate/Nonoxynol-10 Maleate/Acrylates Copolymer; Stearamide; Stearamide DEA-Distearate; Stearamide DIBA-Stearate; Stearamide MEA-Stearate; Stearyl Palmitate; Styrene/Acrylamide Copolymer; Styrne/Acrylates Copolymer; Styrene/Acrylates/Acrylonitrile Copolymer; Styrene/Butadiene Copolymer; Styrene/Isoprene Copolymer; Styrene/Methylstyrene/Indene Copolymer; Styrene/VA Copolymer; Talc; Tallow Amide; Tetradecyloctadecyl Behenate; Tetradecyloctadecyl Myristate; Tetradecyloctadecyl Stearate; Tin Oxide; Titanium Dioxide; Titanium Hydroxide; Titanium Isostearates; Titanium/Titanium Dioxide; Tricalcium Phosphate; Trimagnesium Phosphate; Zinc Carbonate; Zinc Ricinoleate; Zirconium Dioxide; Zirconium Silicate

In one embodiment the composition comprises an anti-dandruff agent. These are known by the one skilled in the art. Examples of useful agents that can be used include: Asarum Heterotropoides Extract; Betula Ermanii Stem Extract; Brassica Campestris (Rapeseed) Flower Extract; Capsicum Annuum Fruit Extract; Carpinus Tschonoskii Leaf Extract; Citronellic Acid; Coal Tar; Coptis Japonica Extract; Fragaria Ananassa (Strawberry) Seed Oil; Hydroxypropyl Bissstearamide MEA; Juniperus Communis Sprout Extract; Lactobacillus/Rice ; Bran/Saccharomyces/Camellia Sinensis Leaf; Extract Ferment; Lactococcus/Bean Seed Extract Ferment Filtrate; Larix Sibirica Wood Extract; Leuconostoc/Radish Root Ferment Filtrate; Magnesium/Aluminum/Zinc/Hydroxide/Carbonate; Mallotus Japonicus Leaf Extract; Morus Bombycis Extract; Octadecenedioic Acid; Peumus Boldus Leaf Oil ; Physocarpus Amurensis Stem Extract; Phytosphingosine Acetamide; Pinus Pinaster Bark/Bud Extract; Piroctone Olamine; Climbazole; Pogostemon Cablin Leaf/Stem Extract; Salicylic Acid; Sciadopitys Verticillata Root Extract; Selenium Sulfide; Smilax China Extract; Sodium Citronellate; Sorbus Aucuparia Seed Oil; Sulfur; Undecylenamidopropyl PEG-2 Dimonium Undecylenate; Vitis Vinifera (Grape) Seed Extract; Zinc Pyrithione (Pyrithione Zinc) ; Zinc Thiosalicylate.

Other useful further ingredients that can be used include:

- salts, such as sodium chloride or ammonium chloride,
- Preservatives,
- Perfumes,
- Fragrances,
- Coloring agents,
- photoprotective agents, UV filters
- emollients,
- pH regulating or adjusting agents, such as citric acid or NaOH
- foam boosting agent
- non aqueous solvents, such as ethanol, isopropanol.

According to one preferred embodiment, the composition is a shampoo.

Process of preparing the composition

The composition can be prepared by any suitable process. Such processes are known by the one skilled in the art. Typical processes involve mixing the different ingredients, with optionally pre-mixing some of them and/or with preparing stock solutions and/or pre-dispersions or the like.

Process of use of the composition

The composition is used to treat hair. The hair treatment is typically preformed by applying the composition onto hair, preferably onto wet hair. After application the hair is usually rinsed. The hair treatment typically provides deposition of the oil onto the hair. With the invention the hair treatment by deposition of the oil onto hair is improved.
The treated hair comprises damaged hair, and optionally virgin hair. According to a preferred embodiment the treated hair comprises damaged hair and virgin hair. This embodiment encompasses the following situations:

- a part of the hair fiber in damaged (damaged part of hair fiber) and another part is not (virgin part of hair fiber), and/or
- some hair fibers are damaged (damaged hair fibers) and some hair fibers are not (virgin hair fiber).

As used herein virgin hair refers to non-damaged hair. Damages of hair can typically be observed by microscopy, preferably with a Scanning Electron Microscope. Typical damaged hair presents with such observation:

- scales, typically standing up scales
- porosity
- discoloration, and/or
- double ends.

Damaged hair can also be characterized by hydrophilic properties, with a contact angle being of lower than 90° for example, typically for chemically damaged hair.

Damage to hair can be the consequence of different causes, including chemical causes, physical causes or other causes.

One common cause of damaged hair can be linked to how one regularly treats its hair, particularly if one uses harsh chemicals. Perms, relaxers, color treatments, and bleach can all be contributing causes of damaged hair. Some of these chemicals cause the cuticles of hair strands to swell, which eventually leaves hair with scales that are rough and apt to chipping or falling off all together. Other chemicals, like perms, relaxers and bleaches are perhaps the more severe causes of damaged hair as they destroy one hair's protein bonds, which weaken the internal structure of the strands. Pollution can also be a cause of damaged hair. The current invention is more particularly efficient for that kind of damage due to chemicals.

Over-grooming hair or using a rough brush or comb can is perhaps one of the most common physical causes of damaged hair. Pillowcases, salt remaining on the hair after an intense workout or swim, and hair accessories can also be physical causes of damaged hair.

Sun rays and heat from hair styling instruments are also common causes of damaged hair. While the sun won't really negatively affect healthy hair, chemically processed hair doesn't respond too well to ultraviolet rays. The sun is often one of the causes of damaged hair because it can cause the hair's protein bonds to diminish and weaken. When heated styling instruments such as curling irons and blow dryers are used too often or on settings that are too hot, they can serve as causes of damaged hair.

The composition of the invention can allow improving the ratio of :

"treatment of damaged hair / treatment of virgin hair."

This ratio is typical of an improved selectivity of the treatment to damaged hair, or of a treatment targeting damaged hair. The selective and/or targeted treatment can be emphasized on communication tools used by suppliers of chemical ingredients of shampoo compositions, for example on animations or movies, presentations, leaflets, flyers, posters, technical data sheets, formularies, on any support, including on papers and websites. This can be linked to a complete or semi complete composition, or to a particular product used to prepare a composition. The selective and/or targeted treatment can as well be emphasized on communication tools used in marketing shampoo compositions, for example on commercial claims, labels, documentation linked to the composition, commercials, scientific studies backing commercial claims, on any support, including on papers, labels, websites, films or animation. Examples of commercial claims can include selective or targeted deposition, selective or targeted treatment, selective or targeted hair repair or the like. Films or animation can for example show a hair fiber (or a representation thereof) having a damaged parts (for example split end or scales) and a product (or a representation thereof) approaching to the hair fiber and treating the damaged part while substantially ignoring the virgin part.

The invention can allow a better targeting of damaged hair. For example it can provide an enhanced deposition of the oil where it is most needed, for example onto tip and end regions. The improved selectivity to damaged hair can allow avoiding drawbacks of intensive and repeated deposition where it is not need, such as build-up effect and/or perception of greasy hair, typically at the root, or perception of heavy hair or dull hair.

The composition of the inventive can provide various treatment benefits such as, take-out, spreadability, rinseability, skin feeling after rinsing, combability of wet hair, feel of wet hair, combability of dry hair and/or feel of dry hair.

## Examples

### Example 1: Compositions

Shampoo compositions detailed on Table 1 are prepared (the amounts are the amounts in wt% "as is" as opposed to amounts as active matter).

Examples 1.2 and 1.3 are comparative examples.

Table 1

| Product Name | INCI name | Example 1.1 | Example 1.2 (Comp.) | Example 1.3 (Comp.) |
|---|---|---|---|---|
| Deionized Water | Aqua | 33.1 | 33.1 | 33.1 |
| Empicol® ESB/3M, Hunstman % Active: 26.5 | Aqua, Sodium Lauryl Ether Sulfate (SLES) | 52.8 | 52.8 | 52.8 |
| Mirataine® BET C30, Rhodia % Active: 30.41 | Aqua, Cocamidoproyl Betaine (CAPB), Sodium Chloride | 6.6 | 6.6 | 6.6 |
| Silicone Emulsion[1] | Aqua, Dimethicone, Laureth-7 | 4.6 | 4.6 | 4.6 |
| Jaguar® C162, Rhodia | Hydroxypropyl Guar Hydroxypropyltrimonium Chloride | 0.3 | | |
| Jaguar® C13S, Rhodia | Guar Hydroxypropyltrimonium Chloride | | 0.3 | |
| Jaguar® C17, Rhodia | Guar Hydroxypropyltrimonium Chloride | | | 0.3 |
| Kathon CG | Magnesium Nitrate, Methyl Chloro Isothiazolinone, Magensium Chloride, Methyl Isothiazolinone | 0.05 | 0.05 | 0.05 |
| NaCl | Sodium Chloride | 1.5 | 1.5 | 1.5 |
| 10% citric acid solution | Aqua, Citric acid | 1 or to pH 5 | 1 or to pH 5 | 1 or to pH 5 |
| [1] Silicone emulsion having an avarage diameter of 0,75 $\mu$m: Dimethicone 65%, Laureth-7 4.1%, Aqua to 100%. | | | | |

Procedure

- In a beaker, weigh water. Start agitation. Add guar and mix during 5 min.
- Adjust pH to 3-4 with citric acid to hydrate guar. Mix during 10-15 min.
- Add CAPB and mix to homogenization
- Add SLES and mix to homogenization
- Add Kathon CG then add the silicone emulsion and mix to homogenization
- Adjust pH to 5 with citric acid.

**Example 2 - Flocculation tests**

Flocculation properties are evaluated on the shampoo compositions of example 1, with the silicone emulsion being replaced with water.

The shampoo compositions are diluted according to the dilution ratios of Table 2.

Transmittance of the diluted compositions is measured at 600 nm with a UV spectrophotometer, using 10x10mm cells.

Table 2

| | Dilution ratio | | | | |
|---|---|---|---|---|---|
| | **2** | **4** | **6** | **8** | **10** |
| **Amount of shampoo (g)** | 5 | 2,5 | 1,67 | 1,25 | 1 |
| **Amount of water (g)** | 5 | 7,5 | 8,33 | 8,75 | 9 |

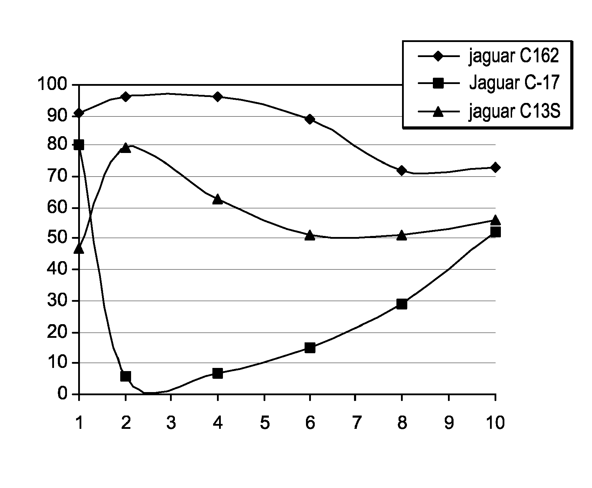

Flocculation properties with the 3 polymers are reported on figure 1 (transmittance at different dilution ratios). The double modified polymer Jaguar® C162 has the worse flocculation profile as flocculation only occurs at high dilution ratios and as the flocculation peak is not high. Thus one expects this polymer to deposit low amounts of silicone oils, compared to comparative polymers.

**Example 3: Evaluation of silicon oil-deposition on virgin & damaged hair and evaluation of selectivity**

The evaluation is performed on the compositions of example 1.
Silicone oil deposition is evaluated on virgin hair virgin as well as on damaged hair with each formulation, by washing using 10 grams of hair approximately with dose of shampoo of 1 gram. The silicone oil deposited on hair is extracted with tetrahydrofuran (THF) and the deposition yield is measured with gas chromatography. The results are given in Table 3 below.
From the deposited amounts one defines the deposition selectivity, by taking the ratio of the amount of silicone deposited on damaged hair, to that deposited on virgin hair:

$$selectivity = \frac{amount\ deposited\ on\ damaged\ hair}{amount\ deposited\ on\ virgin\ hair} \qquad Eq.\ 1$$

Table 3

| Shampoo reference | VIRGIN HAIR | DAMAGED HAIR | selectivity |
|---|---|---|---|
| | ppm silicone $\mu$g/g of hair | ppm silicone $\mu$g/g of hair | |
| Example 1.1 With Jaguar® C162 | 720 | 235 | 33% |
| Example 1.2 (comp.) With Jaguar® C13S | 1044 | 94 | 9% |
| Example 1.3 (comp.) With Jaguar® C17 | 1516 | 66 | 4% |

These results show that contrary to what was expected from the flocculation curves, the double modified polymer allows a better equilibrium between deposition on virgin hair and on damaged hair. This allows a significant treatment of damaged parts of hair while avoiding excess of treatment on virgin parts of hair and can allow reducing build-up negative effects.

**Claims**

1. Use of a double modified cationic guar comprising

   a) cationic modifying groups, and
   b) non ionic modifying groups being $C_3$-$C_{22}$ alkyl or hydroxyalkyl groups,

   in a composition for hair treatment comprising an oil, to increase the selectivity of the treatment for damaged hair.

2. Use according to claim 1, wherein the cationic modifying groups comprise a group of formula -$N^+Me_3$.

3. Use according to claim 1 or 2, wherein the double modified cationic guar is Hydroxypropyl Guar Hydroxypropyltrimonium Chloride.

**4.** Use according to one of claims 1 to 3, wherein the composition comprises a surfactant.

**5.** Use according to claim 4, wherein the composition comprises an anionic surfactant.

**6.** Use according to claim 5, wherein the composition comprises a mixture or an association of an anionic surfactant and an amphoteric surfactant.

**7.** Use according to one of claims 1 to 6, wherein the oil is

- a silicone oil,
- an oil of mineral origin,
- an oil of vegetal origin, or
- a mixture or association thereof.

**8.** Use according to one of claims 1 to 7, wherein the hair to be treated is a chemically damaged hair.

**9.** Use according to one of claims 1 to 8, wherein the composition is a shampoo.


**Patentansprüche**

**1.** Verwendung eines zweifach modifizierten kationischen Guars, umfassend

a) kationische Modifizierungsgruppen und
b) nichtionische Modifizierungsgruppen, die $C_3$-$C_{22}$-Alkyl- oder Hydroxyalkylgruppen sind,

in einer Zusammensetzung für die Haarbehandlung, die ein Öl umfasst, zum Erhöhen der Selektivität der Behandlung für geschädigtes Haar.

**2.** Verwendung gemäß Anspruch 1, wobei die kationischen Modifizierungsgruppen eine Gruppe der Formel -$N^+Me_3$ umfassen.

**3.** Verwendung gemäß Anspruch 1 oder 2, wobei der zweifach modifizierte kationische Guar Hydroxypropylguar-Hydroxypropyltrimoniumchlorid ist.

**4.** Verwendung gemäß einem der Ansprüche 1 bis 3, wobei die Zusammensetzung ein grenzflächenaktives Mittel umfasst.

**5.** Verwendung gemäß Anspruch 4, wobei die Zusammensetzung ein anionisches grenzflächenaktives Mittel umfasst.

**6.** Verwendung gemäß Anspruch 5, wobei die Zusammensetzung ein Gemisch oder eine Vereinigung eines anionischen grenzflächenaktiven Mittels und eines amphoteren grenzflächenaktiven Mittels umfasst.

**7.** Verwendung gemäß einem der Ansprüche 1 bis 6, wobei das Öl

- ein Siliconöl,
- ein Öl mit mineralischem Ursprung,
- ein Öl mit pflanzlichem Ursprung oder
- ein Gemisch oder eine Vereinigung davon

ist.

**8.** Verwendung gemäß einem der Ansprüche 1 bis 7, wobei das zu behandelnde Haar ein chemisch geschädigtes Haar ist.

**9.** Verwendung gemäß einem der Ansprüche 1 bis 8, wobei die Zusammensetzung ein Shampoo ist.

**Revendications**

1.  Utilisation d'une gomme de guar cationique doublement modifiée, comprenant :

    a) des groupes cationiques modificateurs, et
    b) des groupes non ioniques modificateurs constitués par des groupes hydroxyalkyle ou alkyle en $C_3$-$C_{22}$,

    dans une composition de traitement pour les cheveux comprenant une huile, pour augmenter la sélectivité du traitement pour des cheveux abîmés.

2.  Utilisation selon la revendication 1, dans laquelle les groupes cationiques modificateurs comprennent un groupe de formule -$N^+Me_3$.

3.  Utilisation selon la revendication 1 ou 2, dans laquelle la gomme de guar cationique doublement modifiée est l'Hydroxypropyl Guar Hydroxypropyltrimonium Chloride.

4.  Utilisation selon l'une quelconque des revendications 1 à 3, dans laquelle la composition comprend un agent tensioactif.

5.  Utilisation selon la revendication 4, dans laquelle la composition comprend un agent tensioactif anionique.

6.  Utilisation selon la revendication 5, dans laquelle la composition comprend un mélange ou une association d'un agent tensioactif anionique et d'un agent tensioactif amphotère.

7.  Utilisation selon l'une quelconque des revendications 1 à 6, dans laquelle l'huile est :

    - une huile de silicone,
    - une huile d'origine minérale,
    - une huile d'origine végétale, ou
    - un mélange ou une association de celles-ci.

8.  Utilisation selon l'une quelconque des revendications 1 à 7, dans laquelle les cheveux à traiter sont des cheveux abîmés par des produits chimiques.

9.  Utilisation selon l'une quelconque des revendications 1 à 8, dans laquelle la composition est un shampooing.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2773710

- US 4565647 A